**Europäisches Patentamt**

**European Patent Office**

**Office européen des brevets**

(19)

(11) Publication number : **0 372 752 B1**

(12) # EUROPEAN PATENT SPECIFICATION

(45) Date of publication of patent specification :
05.05.93 Bulletin 93/18

(51) Int. Cl.[5] : **A61K 37/02, A61K 47/30**

(21) Application number : **89312131.9**

(22) Date of filing : **22.11.89**

(54) **CD4 polypeptide derivatives.**

(30) Priority : **23.11.88 US 275296**

(43) Date of publication of application :
**13.06.90 Bulletin 90/24**

(45) Publication of the grant of the patent :
**05.05.93 Bulletin 93/18**

(84) Designated Contracting States :
**AT BE CH DE FR GB LI LU NL SE**

(56) References cited :
EP-A- 98 110
WO-A-88/01304
WO-A-89/01940
FR-A- 2 342 305
NATURE, vol. 331, no. 6151, 07 January 1988,
London (GB); R.A.FISHER et al., pp. 76-78*
NATURE, vol. 335, no. 6188, 22 September
1988, London (GB); V.K.CHAUDHARY et al., pp.
369-372*
METHODS IN ENZYMOLOGY, vol. 62, 1979;
E.A.BAYER et al., pp. 308-315*

(73) Proprietor : **GENENTECH, INC.**
**460 Point San Bruno Boulevard**
**South San Francisco California 94080 (US)**

(72) Inventor : **Capon, Daniel J.**
**817 Oregon Avenue**
**San Mateo, CA 94402 (US)**
Inventor : **Shak, Steven**
**1133 Cambridge Road**
**Burlingame, CA 94010 (US)**
Inventor : **Ward, Rebecca H.R.**
**807 E1 Camino Real 1**
**Burlingame, CA 94010 (US)**

(74) Representative : **Armitage, Ian Michael et al**
**MEWBURN ELLIS 2 Cursitor Street**
**London EC4A 1BQ (GB)**

EP 0 372 752 B1

## Description

This application is concerned with derivatized polypeptides, particularly the cell differentiation antigen CD4 and its use as a therapeutic agent for the treatment of HIV infections.

The primary immunologic abnormality resulting from infection by HIV is the progressive depletion and functional impairment of T lymphocytes expressing the CD4 cell surface glycoprotein (H. Lane et al., Ann. Rev. Immunol. 3:477 [1985]). CD4 is a non-polymorphic glycoprotein with homology to the immunoglobulin gene superfamily (P. Maddon et al., Cell 42:93 [1985]). Together with the CD8 surface antigen, CD4 defines two distinct subsets of mature peripheral T cells (E. Reinherz et al., Cell 19:821 [1980]), which are distinguished by their ability to interact with nominal antigen targets in the context of class I and class II major histocompatibility complex (MHC) antigens, respectively (S.Swain, Proc. Natl. Acad. Sci. 78:7101 [1981]; E. Engleman et al., J. Immunol. 127:2124 [1981]; H. Spitz et al., J. Immunol. 129: 1563 [1982]; W. Biddison et al., J. Exp. Med. 156:1065 [1982]; and D. Wilde et al., J. Immunol. 131:2178 [1983]). For the most part, CD4 T cells display the helper/inducer T cell phenotype (E. Reinherz, supra), although CD4 T cells characterized as cytotoxic/suppressor T cells have also been identified (Y. Thomas et al., J. Exp. Med. 154:459 [1981]; S. Meuer et al., Proc. Natl. Acad. Sci. USA 79:4395 [1982]; and A. Krensky et al., Proc. Natl. Acad. Sci. USA 79:2365 [1982]). The loss of CD4 helper/inducer T cell function probably underlies the profound defects in cellular and humoral immunity leading to the opportunistic infections and malignancies characteristic of the acquired immunodeficiency syndrome (AIDS) (H. Lane supra).

Studies of HIV-I infection of fractionated CD4 and CD8 T cells from normal donors and AIDS patients have revealed that depletion of CD4 T cells results from the ability of HIV-I to selectively infect, replicate in, and ultimately destroy this T lymphocyte subset (D. Klatzmann et al., Science 225:59 [1984]). The possibility that CD4 itself is an essential component of the cellular receptor for HIV-I was first indicated by the observation that monoclonal antibodies directed against CD4 block HIV-I infection and syncytia induction (A. Dalgleish et al., Nature [London] 312:767 [1984]; J. McDougal et al., J. Immunol. 135:3151 [1985]). This hypothesis has been confirmed by the demonstration that a molecular complex forms between CD4 and gp120, the major envelope glycoprotein of HIV-I (J. McDougal et al., Science 231:382 [1986]; and the finding that HIV-I tropism can be conferred upon ordinarily non-permissive human cells following the stable expression of a CD4 cDNA (P. Maddon et al., Cell 47:333 [1986]). Furthermore, the neurotropic properties of HIV-I, reflected by a high incidence of central nervous system dysfunction in HIV-I infected individuals (W. Snider et al., Ann. Neurol. 14:403 [1983]), and the ability to detect HIV-I in the brain tissue and cerebrospinal fluid of AIDS patients (G. Shaw et al., Science 227:177 [1985]; L. Epstein, AIDS Res. 1:447 [1985]; S. Koenig, Science 233:1089 [1986]; D. Ho et al., N. Engl. J. Med. 313:1498 [1985]; J. Levy et al., Lancet II:586 [1985]), appears to have its explanation in the expression of CD4 in cells of neuronal, glial and monocyte/macrophage origin (P. Maddon, Cell 47:444 [1986]; I. Funke et al., J. Exp. Med. 165:1230 [1986]; B. Tourvieille et al., Science 234:610 [1986]).

In addition to determining the susceptibility to HIV-I infection, the manifestation of cytopathic effects in the infected host cell appears to involve CD4. Antibody to CD4 was found to inhibit the fusion of uninfected CD4 T cells with HIV-I infected cells in vitro; moreover, the giant multinucleated cells produced by this event die shortly after being formed resulting in the depletion of the population of CD4 cells (J. Lifson et al., Science 232:1123 [1986]). Formation of syncytia also requires gp120 expression, and can be elicited by coculturing CD4-positive cell lines with cell lines expressing the HIV-I env gene in the absence of other viral structural or regulatory proteins (J. Sodroski et al., Nature 322:470 [1986]; J. Lifson et al., Nature 323:725 [1986]). Thus, in mediating both the initial infection by HIV-I as well as eventual cell death, the interaction between gp120 and CD4 constitutes one of several critical entry points in the viral life cycle amenable to therapeutic intervention (H. Mitsuya et al., Nature 325:773 [1987]).

The known sequence of the CD4 precursor predicts a hydrophobic signal peptide, an extracellular region of approximately 370 amino acids, a highly hydrophobic stretch with significant identity to the membrane-spanning domain of the class II MHC beta chain, and a highly charged intracellular sequence of 40 residues (P. Maddon, Cell 42:93 [1985]). The extracellular domain of CD4 consists of four contiguous regions each having amino acid and structural similarity to the variable and joining (V-J) domains of immunoglobulin light chains as well as related regions in other members of the immunoglobulin gene superfamily. These structurally similar regions of CD4 are termed the $V_1$, $V_2$, $V_3$ and $V_4$ domains.

A successful strategy in the development of drugs for the treatment of many receptor mediated abnormalities has been the identification of antagonists which block binding of the natural ligand. Since CD4 ordinarily binds to the recognition sites of the HIV envelope it would appear to be a candidate for therapeutically sequestering these HIV sites, thereby blocking viral infectivity. However, native CD4 is a cell membrane protein which is anchored in the lipid bilayer of cells.

The membrane anchoring property of native CD4 is undesirable from the standpoint of manufacturing, purification and clinical use, and as a result CD4 amino acid sequence variants have been made from which the transmembrane domain has been deleted. These CD4 variants are water soluble and have been demonstrated to suppress HIV replication in *in vitro* cell culture. (See for example Smith *et al.*, "Science" 238: 1704-1707 [1987]; Fisher *et al.*, "Nature" 331:76-78 [1988]; Hussey *et al.*, "Nature" 331:78-82 [1988]; Deen *et al.*, "Nature" 331:82-84 [1988]). Fusions of immunoglobulin light chains with CD4 has been described (Traunecker *et al.*, Nature" 331:84-86 [1988]).

Covalent modification of certain proteins with polyethylene glycol (PEG) or copolymers of polyoxyethylene and polyoxypropylene has been shown to increase *in vivo* plasma half-life, reduce immunogenicity, and/or improve aqueous solubility. See for example, Abuchowski *et al.*, "J. Biol.Chem." 252:3582-3586 [1977]; Abuchowski *et al.*, "Cancer Treatment Rep." 63:1127-1132 [1979]; Pyata *et al.*, "Res. Commun. Chem. Pathol. Pharmacol." 29:113-127 [1980]; Naoi *et al.*, "J. Appl. Biochem." 6:91-102 [1984]; and U.S. 4,609,546. However, covalent modification of proteins with PEG frequently leads to loss of biologic activity. As stated by Harris, "Rev. Macromol. Chem. Phys." 25(3):325-373 (1985), "there appear to be no general rules" with regard to production of excessive protein deactivation, "as effects vary greatly from system to system."

Recent data suggests that lysine residues are likely to play a critical role in gp120 binding. Peterson and Seed found that an eight amino acid segment in CD4 is critical for gp120 binding (aa 42-49; Ser-Phe-Leu-Thr-Lys-Gly-Pro-Ser [Cell, 54:65, 1988]). For example, not only does deletion of this segment abolish gp120 binding, but also substitutions of Asn for Lys46 and Val for Gly47 also abolish gp120 binding. Subsequently, Clayton *et al.* also showed that amino acid substitutions which removed lysine residues in this region of CD4 greatly altered gp120 binding (Nature, 335:363, 1988). For example, substitutions of Pro for Lys50, Ser for Leu51 and Pro for Gly48 abolished gp120 binding.

We have found that truncated forms of CD4 have a short *in vivo* plasma half life in relation to serum protein and an incomplete bioavailability after subcutaneous or intramuscular injection. This is undesirable in the context of CD4 therapy, which may depend on the ready and continuous availability *in vivo* of circulating CD4 to bind to HIV, gp120 of HIV, or HIV-infected cells present in the patient. While it is possible to administer CD4 by continuous infusion, this is inconvenient and expensive for the patient and limits the steady state concentration that can be attained in a patient.

WO 89/01940, prior art only under Art. 54(3)EPC, discloses the production of certain soluble recombinant T4 proteins, and at one point suggests cross-linking them with e.g. polymer to form polyvalent T4 substances. However, there is no specific teaching which would lead thereby to soluble substances with enhanced bioaviability, e.g. improved plasma half-life.

According to one aspect of the present invention there is provided a non proteinaceous polymer conjugated to CD4 whereby a CD4 derivative is prepared that is water soluble and exhibits other desired characteristics. In preferred embodiments the polymer is polyethylene glycol (PEG) which is covalently bonded to an amino group of a CD4 amino acid sequence variant from which the transmembrane region has been deleted.

In another embodiment a nonproteinaceous polymer, particularly PEG, is covalently bound to a carbohydrate substituent of a glycoprotein such as CD4 whereby a water soluble polypeptide derivative is obtained.

CD4 is defined herein as native, full length CD4 or any polypeptide having the qualitative gp120 binding characteristics of native CD4 and comprising a gp120 binding domain of CD4, together with amino acid sequence variants of native CD4 which are capable of binding gp120. CD4 amino acid sequence variants which are useful are readily identified by determining their ability to bind gp120 in accord with analytical methods known *per se* and described in the examples. In general, such variants fall into one or both of two groups. One group comprises CD4 variants in which at least the transmembrane domain is inactivated so that it is incapable of cell membrane insertion. This is typically accomplished by deletion of the transmembrane domain, optionally including deletion of the cytoplasmic domain and all extracellular sequence located downstream from the first two variable region-like domains of CD4. The second group comprises fusions of CD4 with plasma proteins having extended plasma half-lives such as albumin, transferrin, or immunoglobulins. The CD4 polypeptides are lysine N-terminal (the native sequence) or are N-terminated by asparagine or other suitable residues in place of lysine. CD4 variants are described in the parent applications. (WO 89/02922 and EP 314317A)

CD4 is expressed in mammalian recombinant cell culture and isolated from nature as a glycoprotein, which is defined as a polypeptide comprising a carbohydrate substituent. In glycoproteins, the carbohydrate typically is a branched polysaccharide containing fucose, N-acetylglucosamine, galactose, mannose, N-acetylneuraminic acid (sialic acid) and other sugar residues. The carbohydrate is substituted at N-linked glycosylation sites (asp X thr/ser, where X is any residue) or, in other polypeptides, at O-linked sites (typically serine) or at both O and N-linked sites. The carbohydrate composition of truncated CD4 from recombinant CHO cells is as follows (there are two N-linked sites; the proportionate distribution of each sugar between the two sites is not presently known).

### Carbohydrate Composition of rCD4

| Residue | Moles per mole rCD4[a] |
|---|---|
| Fucose | $0.54 \pm 0.03$ |
| N-acetylglucosamine | $6.3 \pm 0.11$ |
| Galactose | $4.0 \pm 0.12$ |
| Mannose | $7.1 \pm 0.25$ |
| N-acetylneuraminic acid | $2.7 \pm 0.05$ |

[a] Mean $\pm$ S.D. (n = 2)

It will be understood that CD4 from other recombinant hosts may contain different sugars or may vary in the relative proportions of the sugars shown above. It is within the scope hereof to move, add or delete glycosylation sites by site-directed mutagenesis of CD4 polypeptide in order to increase the number of or change the location of the carbohydrate-polymer substituents. It also will be understood that the nonproteinaceous polymer which is conjugated to CD4 excludes oligosaccharides that may be present in the native or starting CD4 molecule, i.e. the polymer is extraneous or heterologous to CD4.

The nonproteinaceous polymer ordinarily is a hydrophilic synthetic polymer, i.e., a polymer not otherwise found in nature. However, polymers which exist in nature and are produced by recombinant or in vitro methods are useful, as are polymers which are isolated from nature. Hydrophilic polyvinyl polymers fall within the scope of this invention, e.g. polyvinylalcohol and polyvinylpyrrolidone. Particularly useful are polyalkylene ethers such as polyethylene glycol, polypropylene glycol, polyoxyethylene esters or methoxy polyethylene glycol; polyoxyalkylenes such as polyoxyethylene, polyoxypropylene, and block copolymers of polyoxyethylene and polyoxypropylene (Pluronics); polymethacrylates; carbomers; branched or unbranched polysaccharides which comprise the saccharide monomers D-mannose, D- and L-galactose, fucose, fructose, D-xylose, L-arabinose, D-glucuronic acid, sialic acid, D-galacturonic acid, D-mannuronic acid (e.g. polymannuronic acid, or alginic acid), D-glucosamine, D-galactosamine, D-glucose and neuraminic acid including homopolysaccharides and heteropolysaccharides such as lactose, amylopectin, starch, hydroxyethyl starch, amylose, dextran sulfate, dextran, dextrins, glycogen, or the polysaccharide subunit of acid mucopolysaccharides, e.g. hyaluronic acid; polymers of sugar alcohols such as polysorbitol and polymannitol; heparin; and polyamides such as polyserine or polyalanine. Where the polysaccharide is the native glycosylation or the glycosylation attendant on recombinant expression of CD4, the site of substitution ordinarily is located at other than an N or O-linked glycosylation site of CD4 or the CD4 is an amino acid sequence variant in which an additional or substitute N or O-linked site has been introduced into the molecule. Mixtures of such polymers are employed, or the polymer may be homogeneous. The polymer prior to crosslinking need not be, but preferably is, water soluble, but the final conjugate must be water soluble. In addition, the polymer should not be highly immunogenic when conjugated to CD4, nor should it possess viscosity that is incompatible with intravenous infusion or injection if it is intended to be administered by such routes.

Preferably the polymer contains only a single group which is reactive with CD4. This helps to avoid crosslinking of CD4 molecules. However, it is within the scope herein to optimize reaction conditions to reduce crosslinking, or to purify the reaction products through gel filtration or chromatographic sieves to recover substantially homogeneous derivatives.

The molecular weight of the polymer ranges about from 100 to 500,000, and preferably is about from 1,000 to 20,000. The molecular weight chosen will depend upon the nature of the polymer and the degree of substitution. In general, the greater the hydrophilicity of the polymer and the greater the degree of substitution, the lower the molecular weight that can be employed. Optimal molecular weights will be determined by routine experimentation. Ordinarily, the molecular weight of the CD4-polymer conjugate will exceed about 70,000 although molecules having lesser molecular weights are suitable.

The polymer generally is covalently linked to CD4 through a multifunctional crosslinking agent which reacts with the polymer and one or more amino acid or sugar residues of CD4. However, it is within the scope of this invention to directly crosslink the polymer to the CD4 by reacting a derivatized polymer with CD4, or vice versa. Also within the scope hereof are non-covalent associative complexes of CD4 and the polymer. Such complexes are most conveniently produced by noncovalently associating with CD4 electronegatively charged polymers such as dextran sulfate, heparin, heparan, chondroitin sulfate or other glycosaminoglycans; or amphoteric polymers having electronegative domains. The alkaline pI of CD4 facilitates the formation of such complexes, which are produced by mixing solutions or suspensions of the polymers and CD4, followed by removal of salts or drying in order to accelerate association between the polymer and CD4.

The CD4 covalent crosslinking site is preferably the N-terminal amino group and epsilon amino groups found on lysine residues, although other amino, imino, carboxyl, sulfydryl, hydroxyl or other hydrophilic groups serve as useful sites of substitution on the CD4 molecule. The polymer may be covalently bonded directly to the CD4 antigen without the use of a multifunctional (ordinarily bifunctional) crosslinking agent. Examples of such crosslinking agents include 1,1-bis(diazoacetyl)-2-phenylethane, glutaraldehyde, N-hydroxysuccinimide esters, for example esters with 4-azidosalicylic acid, homobifunctional imidoesters including disuccinimidyl esters such as 3,3′-dithiobis (succinimidyl-propionate), and bifunctional maleimides such as bis-N-maleimido-1,8-octane. Derivatizing agents such as methyl-3-[(p-azido-phenyl)dithio] propioimidate yield photoactivatable intermediates which are capable of forming cross-links in the presence of light. Alternatively, reactive water soluble matrices such as cyanogen bromide activated carbohydrates and the systems described in U.S. patents 3,959,080; 3,969,287; 3,691,016; 4,195,128; 4,247,642; 4,229,537; 4,055,635 and 4,330,440 are suitably modified for cross-linking the polymer and CD4. Covalent bonding to CD4 amino groups is accomplished by known chemistries based upon cyanuric chloride, carbonyl diimidazole, aldehyde reactive groups (PEG alkoxide plus diethyl acetal of bromoacetaldehyde; PEG plus DMSO and acetic anhydride, or PEG chloride plus the phenoxide of 4-hydroxybenzaldehyde) succinimidyl active esters, activated dithiocarbonate PEG, 2,4,5-trichlorophenylchloroformate or p-nitrophenylchloroformate activated PEG. Carboxyl groups are derivatized by coupling PEG-amine using carbodiimide.

Polymers are conjugated to the oligosaccharide substituents by chemical, e.g. metaperiodate, or enzymatic oxidation, e.g. glucose or galactose oxidase, (to produce the aldehyde derivative of the carbohydrate), followed by reaction with hydrazide or amino-derivatized polymers, in the same fashion as is described by Heitzmann *et al.*, P.N.A.S., 71:3537-3541 (1974) or Bayer *et al.*, Methods in Enzymology, 62:310 (1979), for the labeling of oligosaccharides with biotin or avidin. Further, other chemical or enzymatic methods which have been used heretofore to link oligosaccharides and polymers may be suitable. Substituted oligosaccharides are particularly advantageous for CD4 because the carbohydrate substituents are located in the C-terminal region of the extracellular domain and hence are not involved in binding to HIV; this will aid in preserving CD4 activity while achieving other objects herein. Also, since there are fewer substitutions than amino acid sites for derivatization, the oligosaccharide products will be more homogeneous in general. Finally, the CD4 oligosaccharide substituents can be enzymatically modified to remove sugars, e.g. by neuraminidase digestion, prior to polymer derivatization.

The polymer will bear a group which is directly reactive with an amino acid side chain, or the N- or C- terminus of CD4 (see the examples below), or which is reactive with the multifunctional cross-linking agent. In general, polymers bearing such reactive groups are known for the preparation of immobilized proteins. In order to use such chemistries here, one should employ a water soluble polymer otherwise derivatized in the same fashion as insoluble polymers heretofore employed for protein immobilization. Cyanogen bromide activation is a particularly useful procedure to employ in crosslinking polysaccharides to CD4.

"Water soluble" in reference to the conjugate means that the conjugate is soluble in physiological fluids such as blood in an amount which is sufficient to achieve a therapeutically effective concentration. Thus, this excludes matrix-insolubilized CD4 as may be used in affinity chromatography to purify HIV or gp120.

"Water soluble" in reference to the starting polymer means that the polymer or its reactive intermediate used for conjugation is sufficiently water soluble to participate in a derivatization reaction with CD4.

The degree of substitution of CD4 will vary depending upon the number of reactive sites on the protein, whether all or a fragment of CD4 is used, whether the CD4 is a fusion with a protein heterologous to CD4, the molecular weight, hydrophilicity and other characteristics of the polymer, and the particular sites chosen. In general, the CD4 domain of the conjugate is substituted with about from 1 to 10 polymer molecules, while any heterologous sequence which is fused to CD4 may be substituted with an essentially unlimited number of polymer molecules so long as the activity of the CD4 moiety is not significantly adversely affected. The optimal degree of crosslinking is easily determined by an experimental matrix in which the time, temperature and other reaction conditions are varied to change the degree of substitution, after which the ability of the conjugates to bind gp120 and/or inhibit the replication of HIV in vitro in cell culture is determined.

In a preferred embodiment, PEG is crosslinked to CD4 through CD4 lysine residues and the N-terminal amino group. Our own studies, consistent with those of the art described above, suggested that modification of lysine residues is likely to unfavorably affect gp120 binding. Greatly reduced gp120 binding was observed after acetylation of as little as 3 lysine residues of CD4 with acetic anhydride. No gp120 binding was observed after acetylation of 4 lysine residues. Thus, we were surprised to find that lysine residues of CD4 could be successfully modified by polymers such as PEG with only minimal reduction of gp120 binding.

The molecular weight of the conjugated polymer, e.g., PEG ranges about from 500 to 100,000. Molecular

5

weights of 2,000, 5,000 or 20,000 are typical.

The polymer, e.g., PEG is crosslinked to CD4 by a wide variety of methods known *per se* for the covalent modification of proteins with nonproteinaceous polymers such as PEG. Certain of these methods, however, are not preferred for the purposes herein. Cyanuric chloride chemistry leads to many side reactions, including protein cross-linking. In addition, it may be particularly likely to lead to inactivation of proteins containing sulfhydryl groups. Carbonyl diimidazole chemistry (Beauchamp *et al.*, "Anal. Biochem." 131:25-33 [1983]) requires high pH (>8.5), which can inactivate proteins. Moreover, since the "activated PEG" intermediate can react with water, a very large molar excess of "activated PEG" over protein is required. For example, we found that conversion of 80% of recombinant CD4 to CD4-PEG using carbonyl diimidazole chemistry required a 1000 fold molar excess of "activated PEG", and a 12 h incubation. The high concentrations of PEG required for the carbonyl diimidazole chemistry also led to problems with purification, as both gel filtration chromatography and hydrophobic interaction chromatography are adversely effected. In addition, we have found that the high concentrations of "activated PEG" precipitates CD4 leading to reduced covalent modification, a problem that *per se* has been noted previously (Davis, U.S. Patent 4,179,337). On the other hand, aldehyde chemistry (Royer, U.S. Patent 4,002,531) is more efficient since it requires only a 40 fold molar excess of PEG and a 1-2 hr incubation. However, the manganese dioxide suggested by Royer for preparation of the PEG aldehyde is problematic "because of the pronounced tendency of PEG to form complexes with metal-based oxidizing agents" (Harris *et al.*, "J. Polym. Sci., Polym. Chem. Ed." 22:341-352 [1984]). Our use of a moffatt oxidation, utilizing DMSO and acetic anhydride, obviates this problem. In addition, the sodium borohydride suggested by Royer must be used at a high pH and has a significant tendency to reduce disulphide bonds. In contrast, we have used sodium cyanoborohydride, which is effective at neutral pH and has very little tendency to reduce disulphide bonds.

The conjugates of this invention are separated from unreacted starting materials by gel filtration. They are further purified by adsorption onto anti-CD4 (OKT4 monoclonal antibodies) or an immobilized gp120 matrix, the latter having the advantage that it only binds conjugates in which the degree or site of substitution has not resulted in the inactivation of gp120 binding. PEG-substituted CD4 is further purified by hydrophobic interaction chromatography. Most conveniently, the conjugates are eluted from the hydrophobic chromatography medium, e.g. alkyl Sepharose, by the use of a decreasing salt gradient. This, as well as the gel filtration approach described above, resolves the conjugates on the basis of the degree of substitution so that it is possible to obtain CD4-PEG which is substantially homogeneous in its degree of molar substitution by PEG, e.g., monosubstituted or disubstituted CD4 which are essentially free of disubstituted or monosubstituted CD4, respectively. The CD4 derivatives herein also are purified in most cases by ion exchange chromatography (adsorption of CD4 to a cation exchange resin, followed by elution, or adsorption of contaminants to an anion exchange resin).

The conjugates of this invention are formulated into physiologically acceptable carriers and sterile filtered for therapeutic use. The concentration of CD4 in therapeutic formulations is not critical, but is typically about from 1 to 20 mg/ml. The conjugates optionally contain a nonionic detergent such as Tween 20 or 80, salts, buffers and other excipients. They are stored as aqueous solutions or lyophilized.

The conjugates are administered by subcutaneous, intramuscular, intravenous or intracerebrospinal injection, intrapulmonary or intranasal aerosols, dermal patches, intravesicular infusion, or the like. The dosage will be determined in accord with clinical practice, but an initial dose will be about from 10 to 300 µg/kg/1-3 times per week. One advantage of the conjugates herein is that they are infrequently administered and do not need to be continuously infused in order to maintain therapeutic dosages *in vivo*.

Other therapies for HIV are employed in concert with the conjugates herein, for example underivatized soluble CD4, AZT, DDC, neutralizing antibodies, immunocytotoxins, gp120 fragments and HIV vaccines.

## Example 1

### Aldehyde chemistry for the preparation of CD4-PEG

### A. Preparation of PEG-aldehyde.

Oxidation of PEG derivatives (methoxy PEG 2000, methoxy PEG 5000, and PEG 20,000) to the aldehydes was performed using the Swern modification of the Moffatt oxidation procedure. Briefly, a mixture of the required PEG derivative (Sigma, 10-40 mmol) and dry DMSO (Aldrich, 5-6 x vol/weight) was warmed to 50-60°C to effect dissolution of the PEG solid, was allowed to cool to near room temperature, and was added to acetic anhydride (0.5 ml, 50 mmol) in dry DMSO (30 ml) under nitrogen. After stirring for 30-40 h at room temperature, the mixture was poured into 5 volumes of anhydrous ether. For PEG 20,000, addition of the ether followed by vigorous mixing was sufficient to cause precipitation of the product. For the methoxy PEG derivatives, addition of the ether followed by vigorous mixing produced an oily emulsion. Addition of ethyl acetate (approximately 100 ml) caused precipitation of the product. The precipitates were collected by filtration through a sintered glass funnel, were washed one time with ether, and then were dried overnight under a vacuum. Synthesis of the aldehyde derivatives was confirmed using Schiff's test.

### B. Preparation of CD4-PEG.

The PEG aldehydes were covalently linked to CD4 by reductive methylation of amino groups using sodium cyanoborohydride. Briefly, recombinant CD4 (rCD4) (asparagine N-terminal CD4 truncated at amino acid residue 368; 40 $\mu$M) was incubated with PEG aldehyde (1-4 mM) and sodium cyanoborohydride (Sigma, 20 mM) in 0.1 M HEPES buffer, pH 7.2, for 2 h at 37°C. Under these conditions, the CD4 molecule was substituted with about 1-4 PEG residues. Alternatively, higher concentrations of PEG aldehyde (for example, 9.5 mM) lead to a CD4 molecule substituted with about 1-10 PEG residues.

### C. Purification of CD4-PEG.

CD4-PEG was purified free of residual PEG aldehyde and unmodified rCD4 by column chromatography. Gel filtration chromatography was performed using AcA34 resin in a 2.6 x 100 cm column using phosphate-buffered saline (PBS) at a flow rate of 0.3 ml/min. Reaction mixtures are directly loaded on the column without dilution or further workup.

Hydrophobic interaction chromatography was performed using phenyl sepharose resin in a HR 5/5 FPLC column (Pharmacia). Saturated ammonium sulfate was added to reaction mixtures to achieve a final concentration of 1.2 and was loaded on a column equilibrated in 1.2 ammonium sulfate/one-third phosphate-buffered saline (pH 7.2). The column was eluted by a gradient of decreasing ammonium sulfate concentration. Under these conditions, rCD4 flows through the column. CD4-PEG sequentially elutes during the gradient elution in the order of least to most substituted, so that CD4-PEG with varying degrees of substitution are recovered free of other substituted CD4-PEG conjugates. In the following examples the CD4-PEG prepared using rCD4 (Asn N-terminal or Lys N-terminal), PEG (5000 MW) in accord with Example 1B using 9.5 mM PEG aldehyde.

### Example 2

### gp120 binding of CD4-PEG

The ability of rCD4 and CD4-PEG to bind to gp120 is measured in a competition ELISA format. Briefly, rCD4 is coated on 96-well plates by overnight incubation. gp120 is then incubated in various molar concentrations of rCD4 or CD4-PEG, and the reaction mixture is incubated in the rCD4 coated wells. After washing, the amount of gp120 bound to the rCD4-coated wells was determined by incubation with an enzyme-linked anti-gp120 antibody. The ability of rCD4 and CD4-PEG to compete for gp120 binding was determined by this assay. The ability of rCD4 to compete with gp120 (with either an Asn or Lys N-terminal amino acid), when covalently modified with 1-10 PEG molecules was reduced only approximately three fold.

### Example 3

### CD4-PEG half-life

The plasma half-life of rCD4 and CD4-PEG was determined by intravenous injection in New Zealand White rabbits. 100 $\mu$g/kg of CD4-PEG (with either an Asn or Lys N-terminal amino acid) was injected intravenously and plasma CD4 concentrations were determined by ELISA at various times after injection. The terminal half-life of underivatized rCD4 in rabbits was approximately 15 min. In contrast, kinetic studies and analysis of gel filtration eluates demonstrated that CD4-PEG exhibited a greatly prolonged half-life with two principal subpopulations. The terminal half-life of about 60% of the CD4-PEG material was 4-8 hours, while about 20% of the CD4-PEG material had a terminal half-life of 25-50 hours. The longer half-life material was more heavily substituted, but converseley exhibited less gp120 binding. Finally, it was shown that the long half-life CD4-PEG was in the rabbit plasma at one day and still retained the ability to bind the envelope glycoprotein of HIV, gp120.

7

## Example 4

### Dextran or Dextran Sulfate Modified CD4

Dextran or dextran sulfate (2.5g) in water (250 ml), adjusted to pH 10.7 with sodium hydroxide is mixed with cyanogen bromide (0.8g in three aliquots). Following 1 hr of vigorous stirring (pH maintained at 10.5-11 by addition of sodium hydroxide), the mixture is dialyzed against 0.2M sodium carbonate solution, pH 9.0 at 4°C. The activated dextran or dextran sulfate solutions are then incubated to rCD4, and coupling is allowed to proceed over 12-24 hours at 4°C.

## Example 5

### Ficoll Modified CD4

Ficoll (Trade Mark) (M.W. 400,000; 1 g) in water (100 ml) is activated with cyanogen bromide (0.5 g) as described above. The activated Ficoll is then coupled to CD4 at pH7-9 at 4°C for 12-24 hours.

Thus, embodiments of the present invention can provide novel CD4 molecules which exhibit extended biological half-life; also improved ability of CD4 to inhibit the replication of HIV in vivo and in vitro and block gp 120 binding to cell surface CD4; also increased bioavailability of CD4; also improved physicochemical characteristics of CD4, including modified solubility, pI, and molecular weight; also improved immunological characteristics of CD4, in particular reduced immunogenicity so as to prevent adverse immunological reactions and immune mediated clearance.

## Claims

1. A water soluble conjugate of CD4 and a hydrophilic nonproteinaceous polymer which conjugate binds HIV gp120 and possesses one or more enhanced physical, physicochemical, physiological or biological properties relative to native CD4.

2. The conjugate of claim 1 wherein the polymer is polyethylene glycol or methoxypolyethylene glycol.

3. The conjugate of claim 1 wherein the polymer is polyvinyl alcohol, polyvinylpyrrolidone, polyoxyalkylene, or a block copolymer of polyoxyethylene and polyoxypropylene.

4. The conjugate of claim 1 wherein the polymer is other than an oligosaccharide.

5. The conjugate of any of claims 1 to 3 wherein the CD4 is covalently bonded to the polymer.

6. The conjugate of claim 1 wherein the polymer is an oligosaccharide which is covalently bonded to the CD4 antigen at a site other than a native N-linked glycosylation site.

7. The conjugate of claim 5 wherein the polymer is covalently bonded to CD4 through a lysine residue or N-terminal amino group of CD4.

8. The conjugate of claim 4 wherein the CD4 is convalently bonded to the polymer through an oligosaccharide substituent of CD4.

9. The conjugate of any one of the preceding claims which is substituted with about from 1 to 10 moles of polymer.

10. The conjugate of any one of the preceding claims wherein CD4 is free of the CD4 transmembrane domain.

11. The conjugate of claim 10 wherein CD4 comprises an immunoglobulin sequence.

12. A conjugate of CD4 and polyethylene glycol or methoxypolyethylene glycol which possesses a substantially homogeneous molar substitution by polyethylene glycol or methoxypolyethylene glycol.

13. The conjugate of claim 12 which is substituted with about from 1 to 10 moles of polyethylene glycol or methoxypolyethylene glycol per mole of CD4.

14. A pharmaceutical composition comprising a conjugate of any one of the preceding claims in a sterile, physiologically acceptable solution.

15. A method for purifying a conjugate of CD4 and polyethylene glycol, comprising absorbing the conjugate on a hydrophobic interaction resin and eluting the conjugate with a declining concentration of salt.

16. A method for separating conjugates of CD4 and polyethylene glycol (PEG) having various degrees of molar substitution by PEG comprising applying a mixture comprising said conjugates on a hydrophobic interaction resin eluting the conjuages with a declining concentration of salt, and recovering conjuagtes of CD4 and PEG with varying degrees of molar substitution by PEG substantially free of other substituted CD4-PEG conjugates.

17. A method for synthesizing a CD4 conjugate with an aldehyde-substituted nonproteinaceous polymer comprising reductively methylating with cyanoborohydride a reaction mixture containing CD4 and the polymer.

18. A method for convalently cross-linking CD4 to nonproteinaceous polymer which employs a succinimidyl active ester.


**Patentansprüche**

1. Wasserlösliches Konjugat aus CD4 und einem hydrophilen nicht-eiweißartigen Polymer, welches Konjugat HIV-gp120 bindet und bezogen auf natives CD4 eine oder mehrere verstärkte physikalische, physikochemische, physiologische oder biologische Eigenschaften besitzt.

2. Konjugat nach Anspruch 1, worin das Polymer Polyäthylenglykol oder Methoxypolyäthylenglykol ist.

3. Konjugat nach Anspruch 1, worin das Polymer Polyvinylalkohol, Polyvinylpyrrolidon, Polyoxyalkylen oder ein Blockcopolymer aus Polyoxyäthylen und Polyoxypropylen ist.

4. Konjugat nach Anspruch 1, worin das Polymer ein anderes als ein Oligosaccharid ist.

5. Konjugat nach einem der Ansprüche 1 bis 3, worin das CD4 kovalent an das Polymer gebunden ist.

6. Konjugat nach Anspruch 1, worin das Polymer ein Oligosaccharid ist, das an einer anderen Stelle als einer nativen N-gebundenen Glykosylierungsstelle kovalent an das CD4-Antigen gebunden ist.

7. Konjugat nach Anspruch 5, worin das Polymer über einen Lysinrest oder eine N-terminale Aminogruppe von CD4 kovalent an CD4 gebunden ist.

8. Konjugat nach Anspruch 4, worin das CD4 über einen Oligosaccharidsubstituenten von CD4 kovalent an das Polymer gebunden ist.

9. Konjugat nach einem der vorhergehenden Ansprüche, das mit etwa von 1 bis 10 Molen Polymer substituiert ist.

10. Konjugat nach einem der vorhergehenden Ansprüche, worin CD4 frei vom CD4-Transmembranbereich ist.

11. Konjugat nach Anspruch 10, worin CD4 eine Immunoglobulinsequenz aufweist.

12. Konjugat von CD4 und Polyäthylenglykol oder Methoxypolyäthylenglykol, das eine im wesentlichen homogene molare Substitution durch Polyäthylenglykol oder Methoxypolyäthylenglykol besitzt.

13. Konjugat nach Anspruch 12, das mit etwa von 1 bis 10 Molen Polyäthylenglykol oder Methoxypolyäthylenglykol pro Mol CD4 substituiert ist.

14. Pharmazeutische Zusammensetzung, die ein Konjugat nach einem der vorhergehenden Ansprüche in einer sterilen, physiologisch annehmbaren Lösung enthält.

15. Verfahren zum Reinigen eines Konjugats aus CD4 und Polyäthylenglykol, umfassend das Adsorbieren des Konjugats an ein hydrophobes Interaktionsharz und das Eluieren des Konjugats mit einer abnehmenden Salzkonzentration.

16. Verfahren zum Trennen von Konjugaten aus CD4 und Polyäthylenglykol (PEG), die verschiedene Grade an molarer Substitution durch PEG aufweisen, umfassend das Aufbringen einer Mischung, welche die genannten Konjugate umfaßt, auf ein hydrophobes Interaktionsharz, das Eluieren der Konjugate mit einer abnehmenden Salzkonzentration und das Gewinnen der Konjugate aus CD4 und PEG mit variierenden Graden an molarer Substitution durch PEG im wesentlichen frei von anderen substituierten CD4-PEG-Konjugaten.

17. Verfahren zum Synthetisieren eines CD4-Konjugats mit einem aldehydsubstituierten nicht-eiweißartigen Polymer, welches das reduktive Methylieren einer Reaktionsmischung, die CD4 und das Polymer enthält, mit Cyanoborhydrid umfaßt.

18. Verfahren zum kovalenten Vernetzen von CD4 mit einem nicht-eiweißartigen Polymer, bei dem ein Succinimidylaktivester eingesetzt wird.


**Revendications**

1. Conjugué hydrosoluble de CD4 et d'un polymère non protéique hydrophile qui se fixe par conjugaison à HIV gp120 et possède une ou plusieurs propriétés physiques, physico-chimiques, physiologiques ou biochimiques accrues, par rapport au CD4 naturel.

2. Conjugué suivant la revendication 1, dans lequel le polymère est le polyéthylène-glycol ou le méthoxypolyéthylène-glycol.

3. Conjugué suivant la revendication 1, dans lequel le polymère est un polymère d'alcool vinylique, la polyvinylpyrrolidone, un polyoxyalkylène, ou bien un copolymère séquencé de polyoxyéthylène et de polyoxypropylène.

4. Conjugué suivant la revendication 1, dans lequel le polymère est autre qu'un oligosaccharide.

5. Conjugué suivant l'une quelconque des revendications 1 à 3, dans lequel le CD4 est lié par covalence au polymère.

6. Conjugué suivant la revendication 1, dans lequel le polymère est un oligosaccharide qui est lié par covalence à l'antigène CD4 à un site autre qu'un site de glycosylation naturelle par liaison à N.

7. Conjugué suivant la revendication 5, dans lequel le polymère est lié par covalence à CD4 par un résidu lysine ou un groupe amino N-terminal de CD4.

8. Conjugué suivant la revendication 4, dans lequel le CD4 est lié par covalence au polymère par un substituant oligosaccharidique de CD4.

9. Conjugué suivant l'une quelconque des revendications précédentes, qui est substitué avec environ 1 à 10 moles de polymère.

10. Conjugué suivant l'une quelconque des revendications précédentes, dans lequel le CD4 est dépourvu du domaine transmembranaire de CD4.

11. Conjugué suivant la revendication 10, dans lequel le CD4 comprend une séquence d'immunoglobuline.

12. Conjugué de CD4 et de polyéthylène-glycol ou de méthoxypolyéthylène-glycol qui possède une substitution molaire pratiquement homogène par le polyéthylène-glycol ou le méthoxypolyéthylène-glycol.

13. Conjugué suivant la revendication 12, qui est substitué avec environ 1 à 10 moles de polyéthylène-glycol ou de méthoxypolyéthylène-glycol par mole de CD4.

14. Composition pharmaceutique comprenant un conjugué suivant l'une quelconque des revendications précédentes dans une solution stérile, physiologiquement acceptable.

15. Procédé de purification d'un conjugué de CD4 et de polyéthylène-glycol, comprenant l'absorption du conjugué sur une résine d'interaction hydrophobe et l'élution du conjugué avec une concentration décroissante en sel.

16. Procédé pour séparer des conjugués de CD4 et de polyéthylène-glycol (PEG) ayant divers degrés de substitution molaire par le PEG, comprenant l'application d'un mélange renfermant lesdits conjugués sur une résine d'interaction hydrophobe, l'élution des conjugués avec une concentration décroissante de sel, et la séparation des conjugués de CD4 et PEG ayant des degrés variables de substitution molaire par le PEG pratiquement dépourvus d'autres conjugués CD4-PEG substitués.

17. Procédé pour synthétiser un conjugué de CD4 avec un polymère non protéique à substituant aldéhyde, comprenant la méthylation réductrice avec un cyanoborohydrure d'un mélange réactionnel contenant le CD4 et le polymère.

18. Procédé pour la réticulation covalente du CD4 à un polymère non protéique, qui utilise un ester de succinimidyle actif.